(19) Europäisches Patentamt
European Patent Office
Office européen des brevets

(11) EP 2 517 688 B1

(12) EUROPEAN PATENT SPECIFICATION

(45) Date of publication and mention of the grant of the patent:
**12.08.2015 Bulletin 2015/33**

(21) Application number: **11165163.4**

(22) Date of filing: **06.05.2011**

(51) Int Cl.:
***A61K 8/31*** (2006.01) ***A61K 8/46*** (2006.01)
***A61Q 9/04*** (2006.01) ***A61Q 17/00*** (2006.01)
***A61K 8/81*** (2006.01) ***A61K 8/891*** (2006.01)
***A61K 8/92*** (2006.01) ***A61K 8/06*** (2006.01)

(54) **Depilation method and kit**

Enthaarungsverfahren und Kit

Procédé et kit d'épilation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2011 US 479882 P**

(43) Date of publication of application:
**31.10.2012 Bulletin 2012/44**

(73) Proprietor: **The Procter & Gamble Company**
**Cincinnati, OH 45202 (US)**

(72) Inventors:
- **Smith, Charles, Robert**
  **Wiltshire, SN1 3PS (GB)**
- **Simpson, Naomi, Mary**
  **Twickenham, Middlesex TW2 5QB (GB)**
- **Deckner, George, Endel**
  **Cincinnati, OH 45249 (US)**

(74) Representative: **Wilding, Richard Alan**
**Procter & Gamble**
**Technical Centres Limited,**
**Patent Department,**
**Rusham Park,**
**Whitehall Lane,**
**Egham**
**Surrey TW20 9NW (GB)**

(56) References cited:
**WO-A1-2006/021782** **GB-A- 2 327 190**
**US-A1- 2004 219 118**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 517 688 B1

## Description

### FIELD OF THE INVENTION

The present invention relates to a depilatory method and kit.

### BACKGROUND OF THE INVENTION

Depilatory compositions are cosmetic hair removal formulations. They comprise keratin reducing agents, which attack the disulphide bonds in hair to weaken it, such that subsequent gentle scraping and/or wiping completes severance of the hair from the skin and effects hair removal. Commercially, the most common keratin reducing agents are thioglycolates, which are typically formulated at high pH. An unwanted side effect of chemical depilation is that the depilatory composition comes into contact with and must have a relatively long residence time on skin to achieve effective hair removal and this long residence time combined with the alkaline conditions needed for effective hair removal may give rise to skin irritation.

The above problem has been recognized in the art. Reference is made to US 4,401,663 and US 4,424,205, the disclosures of which are similar to one another. These documents teach to use certain compounds as topical analgesics and an example given of a situation in which it is suggested to use such materials is to reduce depilatory irritation. The carrier formulas disclosed to be suitable for formulation with the analgesics include lotions and creams.

Reference is also made to US 2004/0219118, which discloses treatment with a "lipophilic" material before application of a thioglycolate-based reactive depilatory compostion. Lipophilic materials exemplified in this patent application are oils, such as mineral oil. As shown hereinbelow, the present applicants have tested a range of lipophilic materials to determine their ability to prevent thioglycolate penetration and, thereby, their ability to reduce or prevent skin irritation Applicants have surprisingly found that oils, such as mineral oil, have no or a low ability to prevent thioglycolate penetration to the skin. There thus exists a need to develop a pre-treatment composition which better reduces skin irritation.

### SUMMARY OF THE INVENTION

According to a first aspect of the invention, a method of removing hair from skin according to claim 1 is provided.

According to a second aspect of the invention, a depilatory kit according to claim 11 is provided.

### BRIEF DESCRIPTION OF THE DRAWINGS

Fig. 1. is a schematic view of a Franz Cell apparatus.

### DETAILED DESCRIPTION OF THE INVENTION

The protective composition used in the method and comprised within the kit according to the invention comprises a sterically stabilized emulsion having a hydrophilic continuous phase and a hydrophobic dispersed phase. As demonstrated by the Franz Cell test data, below, the applicants have established that such emulsions are significantly better at preventing the penetration of keratin reducing agent, such as thioglycolate, to the skin, than oil on its own or than emulsions stabilized by traditional surfactants, and therefore result in reduced skin irritation. In addition, applicants have established that the use of the present protective composition still permits hair weakening and destruction by the keratin reducing agent, so that reduced skin irritation can be achieved while removing hair.

It is not fully understood why the present protective composition provides such an effective barrier to penetration of keratin reducing agents. Without wishing to be bound by theory, it is believed that the present, sterically stabilized emulsions may provide a better barrier than traditional emulsions, because the steric emulsifiers used herein are large molecules which are relatively immobile when compared with traditional surfactants, This factor diminishes their detergent properties, thereby reducing or even eliminating their tendency to act to pull the hydrophobic phase off the skin following application and re-emulsify it. Removal of the hydrophobic phase which provides the barrier against thioglycolate penetration would increase such penetration giving a correspondingly lower reduction in irritation than might otherwise be achieved.

A reduction of thioglycolic acid penetration of 45% or more according to the Franz Cell test method may be shown to correlate to a significant and user-noticeable reduction in irritation.

The protective composition used in the method and comprised within the kit according to the invention comprises an emulsion having a hydrophilic continuous phase. Advantageously, the protective composition comprises from 15% to 80%, preferably from 20% to 70%, more preferably from 25% to 65% hydrophilic continuous phase by weight of the

protective composition.

The hydrophilic continuous phase typically comprises water, but need not. In addition to or as an alternative to water, the hydrophilic continuous phase may comprise hydrophilic materials, such as polyhydric alcohols, ethoxylated and propoxylated polyols, polysaccharides, and mixtures thereof. Suitable polyhydric alcohols (polyols) include, but are not limited to, butylene glycol, hexylene glycol, ethoxydiglycol, dipropylene glycol, phenyl ethyl alcohol, glycerin, 1,3-butanediol, 1,2-propanediol, isoprene glycol, sorbitol, polyethylene glycol, polypropylene glycol and mixtures thereof. Preferred polyols include glycerin, propylene glycol, panthenol and mixtures thereof.

Additionally, the hydrophilic continuous phase may comprise other polar solvents, such as alcohols, ketones and mixtures thereof. Examples of suitable polar solvents include phenyl ethyl alcohol, ethanol, isopropyl alcohol and mixtures thereof.

The hydrophilic continuous phase may additionally comprise hydrophilic skin active agents such as, but not limited to, vitamins, including hydrophilic ascorbic acid compounds and vitamin B3 compounds; azelaic acid; gallic acid and its derivatives; N-acetyl glucosamine; panthenol and mixtures thereof.

The protective composition used in the method and comprised within the kit according to the invention comprises an emulsion having a hydrophobic dispersed phase. Advantageously, the protective composition comprises from 20% to 85%, preferably from 20 to 75%, more preferably from 35% to 70% hydrophobic dispersed phase by weight of the protective composition.

The hydrophobic dispersed phase may comprise one or more oils. As used herein, the term "oil" includes, but is not limited to any non-aqueous substance that is practically insoluble in water according to the United States' Pharmacopeia (USP) definition in 31/NF 26 Vol. 2 General Notices, Page Xvii. (which, according to that definition, means that more than 10,000 parts of water are needed to dissolve 1 part solute) and is liquid at 20°C.

The oil may be selected from natural oil, synthetic oil, silicone oil and mixtures thereof.

Non-limiting examples of suitable natural oils include Acetylated Castor Oil, Acetylated Hydrogenated Castor Oil, Actinidia Chinensis (Kiwi), Seed Oil, Adansonia Digitata Oil, Aleurites Moluccana Seed Oil, Anacardium Occidentale (Cashew) Seed Oil, Arachis Hypogaea (Peanut) Oil, Arctium Lappa Seed Oil, Argania Spinosa Kernel Oil, Argemone Mexicana Oil, Avena Sativa (Oat) Kernel Oil, Bertholletia Excelsa Seed Oil, Borago Officinalis Seed Oil, Brassica Campestris (Rapeseed) Seed Oil, Calophyllum Tacamahaca Seed Oil, Camellia Japonica Seed Oil, Camellia Kissi Seed Oil, Camellia Oleifera Seed Oil, Canola Oil, Caprylic/Capric/Lauric Triglyceride, Caprylic/Capric/Linoleic Triglyceride, Caprylic/Capric/Myristic/Stearic Triglyceride, Caprylic/Capric/Stearic Triglyceride, Caprylic/Capric Triglyceride, Carthamus Tinctorius (Hybrid Safflower) Seed Oil, Carthamus Tinctorius (Safflower) Seed Oil, Carum Carvi (Caraway) Seed Oil, Carya Illinoensis (Pecan) Seed Oil, Castor Oil Benzoate, Chenopodium Quinoa Seed Oil, Cibotium Barometz Oil, Citrullus Vulgaris (Watermelon) Seed Oil, Cocos Nucifera (Coconut) Oil, Cod Liver Oil, Coffea Arabica (Coffee) Seed Oil, Coix Lacryma-Jobi (Job's Tears) Seed Oil, Corylus Americana (Hazel) Seed Oil, Corylus Avellana (Hazel) Seed Oil, Cucumis Sativus (Cucumber) Oil, Cucurbita Pepo (Pumpkin) Seed Oil, Daucus Carota Sativa (Carrot) Seed Oil, Elaeis Guineensis (Palm) Kernel Oil, Elaeis Guineensis (Palm) Oil, Gossypium (Cotton) Seed Oil, Helianthus Annuus (Hybrid Sunflower) Oil, Helianthus Annuus (Sunflower) Seed Oil, Hippophae Rhamnoides Oil, Human Placental Lipids, Hydrogenated Canola Oil, Hydrogenated Castor Oil, Hydrogenated Castor Oil Laurate, Hydrogenated Castor Oil Triisostearate, Hydrogenated Coconut Oil, Hydrogenated Cottonseed Oil, Hydrogenated C12-18 Triglycerides, Hydrogenated Fish Oil, Hydrogenated Lard, Hydrogenated Menhaden Oil, Hydrogenated Mink Oil, Hydrogenated Olive Oil, Hydrogenated Orange Roughy Oil, Hydrogenated Palm Kernel Oil, Hydrogenated Palm Oil, Hydrogenated Peanut Oil, Hydrogenated Rapeseed Oil, Hydrogenated Shark Liver Oil, Hydrogenated Soybean Oil, Hydrogenated Sunflower Seed Oil, Hydrogenated Tallow, Hydrogenated Vegetable Oil, Isatis Tinctoria Seed Oil, Juglans Regia (Walnut) Seed Oil, Lauric/Palmitic/Oleic Triglyceride, Umnanthes Alba (Meadowfoam) Seed Oil, Unum Usitatissimum (Linseed) Seed Oil, Lupinus Albus Seed Oil, Macadamia Integrifolia Seed Oil, Macadamia Ternifolia Seed Oil, Maleated Soybean Oil, Mangifera Indica (Mango) Seed Oil, Marmot Oil, Melaleuca Alternifolia (Tea Tree) Leaf Oil, Melia Azadirachta Seed Oil, Melissa Officina lis (Balm Mint) Seed Oil, Menhaden Oil, Mink Oil, Moringa pterygosperma Seed Oil, Mortierella Oil, Neatsfoot Oil, Nelumbium Speciosum Flower Oil, Nigella Sativa Seed Oil, Oenothera Biennis (Evening Primrose) Oil, Olea Europaea (Olive) Fruit Oil, Olea Europaea (Olive) Husk Oil, Orange Roughy Oil, Orbignya Cohune Seed Oil, Orbignya Oleifera Seed Oil, Oryza Sativa (Rice) Bran Oil, Oryza Sativa (Rice) Germ Oil, Ostrich Oil, Oxidized Corn Oil, Oxidized Hazel Seed Oil, Papaver Orientale (Poppy) Seed Oil, Passiflora Edulis Seed Oil, Persea Gratissima (Avocado) Oil, Pistacia Vera Seed Oil, Placental Lipids, Prunus Amygdalus Amara (Bitter Almond) Kernel Oil, Prunus Amygdalus Dulcis (Sweet Almond) Oil, Prunus Armeniaca (Apricot) Kernel Oil, Prunus Avium (Sweet Cherry) Seed Oil, Prunus Cerasus (Bitter Cherry) Seed Oil, Prunus Persica (Peach) Kernel Oil, Pyrus Malus (Apple) Oil, Ribes Nigrum (Black Currant) Seed Oil, Ricinus Communis (Castor) Seed Oil, Rosa Canina Fruit Oil, Rosa Moschata Seed Oil, Salmon Oil, Salvia Hispanica Seed Oil, Santalum Album (Sandalwood) Seed Oil, Sesamum Indicum (Sesame) Seed Oil, Shark Liver Oil, Solanum Lycopersicum (Tomato) Seed Oil, Soybean Lipid, Sphingolipids, Taraktogenos Kurzii Seed Oil, Telphairia Pedata Oil, Vegetable Oil, Vitis Vinifera (Grape) Seed Oil, Zea Mays (Corn) Germ Oil, Zea Mays (Corn) Oil and mixtures thereof.

Non-limiting examples of suitable synthetic oils include mineral oil, isopropyl pamitate, isopropyl stearate, isohexadecane, isododecane, polyglyceryl triisostearate and mixtures thereof.

Non-limiting examples of suitable silicone oils include dimethicones (including partial esters of dimethicones and fatty acids derived from natural/synthetic oils), cyclomethicones, polydimethlysiloxanes, phenyl trimethicones, trimethyl pentaphenyl trisiloxane, dimethicone copolyols and mixtures thereof. Advantageously, the hydrophobic continuous phase of the emulsion comprises dimethicone having a viscosity of 1 to 10,0000 centistoke, preferably 5 to 1000 centistoke, more preferably 25 to 500 centistoke.

Non-limiting examples of commercially available silicone oils include Dow Corning 200 fluid, Dow Corning 244, Dow Corning 245, Dow Corning 344, and Dow Corning 345, (commercially available from Dow Corning Corp.); SF-1204 and SF-1202 Silicone Fluids (commercially available from G.E. Silicones), GE 7207 and 7158 (commercially available from General Electric Co.); and SWS-03314 (commercially available from SWS Silicones Corp.), the Viscasil series (sold by General Electric Company), SF 1075 methyl-phenyl fluid (sold by General Electric Company) and 556 Cosmetic Grade Fluid (sold by Dow Corning Corp.), SF1066 organosilicone surfactant (sold by General Electric Company). Advantageously, where there is a choice, these oils will be selected to have a viscosity within the above-defined ranges.

The protective composition may comprise from 0.75% to 15%, preferably from 1% to 10%, more preferably from 1% to 8% wax by weight of the protective composition.

As used herein, the term "wax" includes, but is not limited to, any hydrophobic material that is:

- Solid at 25°C
- practically insoluble in water according to the United States' Pharmacopeia (USP) definition in 31/NF 26 Vol. 2 General Notices, Page Xvii. (which, according to that definition, means that more than 10,000 parts of water are needed to dissolve 1 part solute);
- has an onset temperature measured according to the DSC Method, defined hereinbelow, which is 10°C or greater; and
- comprises lipids, silicones or mixtures thereof.

The wax may comprise natural wax, synthetic wax, silicone wax, or mixtures thereof.

Non-limiting examples of suitable natural waxes include Abies Alba Leaf Wax, Acacia Dealbata Leaf Wax, Acacia Farnesiana Flower Wax, Beeswax, Ceresin, Cetyl Esters, Cistus Labdaniferus Flower Wax, Aurantium Amara (Bitter Orange) Flower Wax, Aurantium Dulcis (Orange) Peel Wax, Copernicia Cerifera (Carnauba) Wax, Eclipta Prostrata Wax, Euphorbia Cerifera (Candelilla) Wax, Helichrysum Angustifolium Wax, Jasminum Officina le (Jasmine) Flower Wax, Jasminum Sambac (Jasmine) Flower Wax, Jojoba Esters, Jojoba Wax, Lanolin Wax, Lavandula Angustifolia (Lavender) Flower Wax, Lawsonia Inermis Wax, Mink Wax, Montan Acid Wax, Montan Wax, Myrica Cerifera (Bayberry) Fruit Wax, Ocimum Tenuiflorum Wax, Olive Wax, Oryza Sativa (Rice) Bran Wax, Ouricury Wax, Palm Kernel Wax, Persea Gratissima (Avocado) Wax, Pistacia Lentiscus Leaf Wax, Polianthes Tuberosa Flower Wax, Pyrus Malus (Apple) Peel Wax, Ribes Nigrum (Black Currant) Wax, Rosa Centifolia Flower Wax, Salvia Sclarea (Clary) Wax, Shellac Wax, Simmondsia Chinensis (Jojoba) Butter, Soft Olive Wax, Spent Grain Wax, Stipa Tenacissima Wax, Sunflower Seed Wax, Vegetable Wax, Vitis Vinifera (Grape) Leaf Wax and mixtures thereof.

Non-limiting examples of suitable synthetic waxes include Hydrogenated Japan Wax, Hydrogenated Jojoba Oil, Hydrogenated Jojoba Wax, Hydrogenated Microcrystalline Wax, Hydrogenated Rice Bran Wax, Hydrolyzed Beeswax, Microcrystalline Wax, Oxidized Beeswax, Oxidized Microcrystalline Wax, Ozokerite, Paraffin, PEG-6 Beeswax, PEG-8 Beeswax, PE G-12 Beeswax, PEG-20 Beeswax, PEG-12 Carnauba, Potassium Oxidized Microcrystalline Wax, Sulfurized Jojoba Oil, Synthetic Beeswax, Synthetic Candelilla Wax, Synthetic Carnauba, Synthetic Japan Wax, Synthetic Jojoba Oil, Synthetic Wax and mixtures thereof.

Non-limiting examples of suitable silicone waxes include DC2503 Cosmetic Wax, DC580 wax, DC AMS-C30 Cosmetic Wax, C30-45 Alkyl Methicone, DC Silkywax 10, Hexamethyldisiloxane, DC ST-Wax 30, C30-45 Alkyldimethylsilyl Polypropylsilsesquioxane, DC SW-8005 resin wax, C26 - 28 Alkyl Dimethicone, C26 - 28 Alkyl Methicone, Polyphenylsilsesquioxane and mixtures thereof.

Advantageously, the wax comprises beeswax, carnauba wax, candelilla wax, jojoba wax, paraffin wax, microcrystalline wax, ozokerite, arachidyl behenate, or mixtures thereof.

The presence of some wax in the hydrophobic dispersed phase of the protective composition may surprisingly further reduce penetration by thioglycolic acid in comparison with a dispersed phase comprising oils alone, while not diminishing depilation efficacy. Without wishing to be bound by theory, applicants believe that this additional effect may be because the wax militates against the tendency otherwise exhibited by oils to ball up on skin and therefore disrupt the barrier. The presence of wax may also ensure that a thin barrier of the protective composition can be evenly distributed across the skin, even at a low dosage per unit area. The wax may form a substantive barrier across the skin (enhanced by an amorphous mix of the oil & wax) that is chemically resistant to ingress from the thioglycolate (or other reducing) actives, therefore physically reducing the ability for the harsh chemistry to come into contact with the skin. This reduction in contact means that the stratum corneum may be maintained in a better state than if no barrier were present with correspondingly reduced signs of irritation, such as erythema, tingling and stinging.

At the same time as reducing contact between the depilatory active ingredient and the skin, the present compositions still permit the depilatory composition to attack and degrade the unwanted hair growing on that skin to achieve hair removal. Why this should be is not fully understood, but it may simply be due to the fact that less of the protective composition adheres to the hairs than to the skin.

The hydrophobic continuous phase may comprise one or more triglycerides, the or each triglyceride having the following formula:

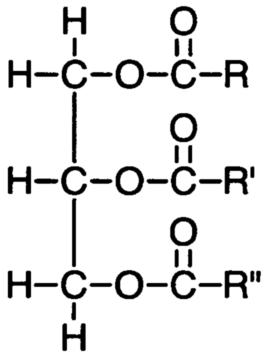

wherein R, R' and R" may be the same as or different from one or both of the others, wherein each of R, R' and R" is a fatty acid and wherein the or each triglyceride is solid at 25°C

Advantageously, the or each triglyceride has an onset temperature of less than 65°C as measured by Differential Scanning Calorimetry. At and above an onset temperature of 65°C, the composition may become increasingly difficult to apply and may, in addition, crack and fall off in use.

Suitable oils from which triglycerides may be formed from include, but are not limited to, the oils listed herein. Suitable fatty acids for formation of triglycerides include, but are not limited to, Myristoleic acid, Palmitoleic acid, Sapienic acid, Oleic acid, Linoleic acid, $\alpha$-Linolenic acid ,Arachidonic acid , Eicosapentaenoic acid, Docosahexaenoic acid, Lauric acid ($C_{12}$), Myristic acid ($C_{14}$), Palmitic acid ($C_{16}$), Stearic acid ($C_{18}$), Arachidic acid ($C_{20}$) and mixtures thereof.

Specific sources of triglycerides suitable for inclusion in the protective composition include include Butter, Shea Butter, Butyrospermum Parkii, Lipex Shea, Theobroma Cacao (Cocoa) Seed Butter, Cocoa Butter, Hydrogenated Shea Butter, Hydrogenated Cocoa Butter, Irvingia Gabonensis Kernel Butter, Tallow, Lard, Mangifera Indica (Mango) Seed Butter, Kokum Butter and mixtures thereof. The triglyceride(s) may fall under the definition of "wax" or "oil" as used herein and, in such a case, should be included as a wax or oil for the purposes of determining the proportions of wax or oil.

The hydrophobic dispersed phase may comprise skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof. These materials may fall under the definition of "wax" or "oil" as used herein and, in such a case, should be included as a wax or oil for the purposes of determining the proportions of wax or oil.

The protective composition used in the method and comprised within the kit according to the invention may include further ingredients such as, but not limited to metal oxides, organic and inorganic dyes, lakes, micas, flavourings, perfumes and mixtures thereof.

The protective composition used in the method and comprised within the kit according to the invention comprises a steric emulsifier, which sterically stabilises the emulsion, such that surfactants are not required. As used herein, the term "steric emulsifier" means an amphipathic polymer which comprise a hydrophilic backbone and hydrophobic side chains. Advantageously, the amphipathic polymer comprises from 0.5 to 5%, preferabily from 1% to 3.5% of hydrophobic side chains by weight of the amphipathic polymer.

Advantageously, the steric emulsifier is a sparingly soluble or less than sparingly soluble in water. The term "sparingly soluble" in water is defined in the United States' Pharmacopeia (USP) definition in 31/NF 26 Vol. 2 General Notices, Page Xvii. (according to that definition, "sparingly soluble" means that 30-300 parts of water are needed to dissolve 1 part solute).

Small amounts of surfactants may be present in the protective composition, but they are preferably absent. As used herein, a "surfactant" is an amphipathic material that is not a steric emulsifier. Surfactants may be present in the following amounts:

(a) Surfactants having an HLB from 1 to 6 may be present in an amount of less than or equal to 3%, preferably less than or equal to 1.5% and more preferably 0% by weight of the protective composition;

(b) Surfactants having an HLB from 7 to 18 may be present in an amount of less than or equal to 0.5%, preferably

0% by weight of the protective composition.

The HLB (the "Hydrophilic-Lipophilic Balance") value system is fully described, and values for various materials are provided, in the publication The HLB System, A Time-Saving Guide to Emulsifier Selection (published by ICI Americas Inc., Wilmington, Del.; 1984). Advantageously, the protective composition comprises from 0.01 % to 5%, preferably from about 0.05% to about 3%, more preferably from 0.05% to 1% by weight of steric emulsifer. The steric emulsifier comprises a copolymer comprising a monomeric mixture comprising:

(a) 95.9% to 98.8% by weight of the copolymer of an olefinically unsaturated carboxylic monomer having the following structure:

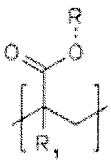

wherein R is hydrogen, methyl or ethyl and $R_1$ is a substituent selected from the class consisting of hydrogen, halogen, and the cyanogen (-C=N) groups, monovalent alkyl radicals, monovalent alkaryl radicals and monovalent cycloaliphatic radicals. Within this class, acrylic, methacrylic, and ethacrylic acid monomers are preferred. Another suitable olefinically unsaturated carboxylic monomer is maleic anhydride or maleic acid.

(b) from 1% to 3.5% by weight of the copolymer of an acrylate ester of the above structural formula, wherein R is an alkyl radical containing 10 to 30 carbon atoms and $R_1$ is hydrogen, methyl or ethyl;

(c) less than or equal to 0.6%, preferably from 0.1% to 0.6% by weight of the copolymer of a polymerizable cross-linking polyalkenyl polyether of a polyhydric alcohol containing more than one alkenyl ether group per molecule of the polyether, wherein the parent polyhydric alcohol contains at least 3 carbon atoms and at least 3 hydroxyl groups.

Advantageously, the copolymer comprises:

(a) from 96% to 97.9% by weight of the copolymer of acrylic acid;
(b) from 2.5% to 3.5% by weight of the copolymer of acrylic ester, wherein the alkyl group contains 12 to 22 carbon atoms and $R_1$ is methyl;
(c) from 0.2% to 0.4% of cross-linking monomer by weight of the copolymer, the corss-linking monomer preferably being selected from the group consisting of allyl pentaerythritol, trimethylolpropane diallylether and allyl sucrose.

Commercially available examples of polymers within this class include Carbomer 1342 (available as Carbopol 1342 from Lubrizol), Carbomer 1382 (Carbopol 1382 from Lubrizol), Carbopol® Ultrez 21 polymer, Carbopol® Ultrez 20, Pemulen TR-1 & Pemulen TR-2 (available from Lubrizol).

Or the steric emulsifier comprises alkyl substituted hydroxyethyl cellulose ether copolymers of the structure:

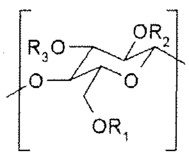

Alkyl Modifier attached via Ether linkage

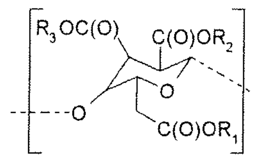

Alkyl Modifier attached via Ester linkage

Whereby a long chain alkyl modifier group having 8 to 25 carbon atoms can be attached to the cellulose ether substrate via an ether or ester linkage (typically attached at position $R_1$), the remaining R-groups being either H, methyl or ethyl. The monomer containing the long chain hydrocarbon group should be present in an amount of about 0.1% to 4.0% by weight of the copolymer, with the remaining monomers having $R_1$, $R_2$ & $R_3$ groups of H, methyl or ethyl. Commercially available examples of polymers within this class include Natrosol Plus 330, Natrosol B and Natrosol (available from Aqualon Ashland), most preferably Natrosol Plus 330.

The protective composition used in the method and comprised within the kit according to the invention may

include further ingredients such as, but not limited to metal oxides, organic and inorganic dyes, lakes, micas, flavourings, perfumes and mixtures thereof.

Advantageously, the keratin reducing agent is comprised within the depilatory composition in an amount from 0.3% to 20%, preferably from 0.8% to 15%, more preferably from 1% to 10% by weight of the depilatory composition. The depilatory composition comprises at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium,

tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.75 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide, sodium hydroxide or mixtures thereof.

In an advantageous embodiment, the base is present at a concentration of from 0.1% to 10.0%, more preferably from 0.5% to 8.0% and even more preferably from 1.0% to 5.0%, by weight of the depilatory composition.

The concentration of water in the depilatory composition is preferably at least 40%, more preferably from 50% to 98%, even more preferably from 60% to 95% and even more preferably still from 70% to 90%, by weight of the depilatory composition.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. The thickening agent may be present at a level of from about 0.01% to about 20%, preferably from about 0.1% to about 10% by weight of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone, including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in cosmetic compositions, such as dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

The depilatory composition may be formulated in any common delivery form, such as a cream or lotion. Alternatively, it may be delivered on a substrate, such as a thin film of depilatory composition coated onto the substrate. The substrate may be configured in any suitable form, such as a strip, mask or patch.

In addition to the protective composition and the depilatory composition, the kit according to the second aspect of the invention may comprise one or more of:

(a) A make-up removal composition and/or a make-up removal wipe;

(b) Means for removal of the protective composition and the depilatory composition following use, which means may comprise one or more of a tool, such as a scraper or a spatula; or a wipe;

(c) A post-treatment composition skin care composition to be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

(d) Instructions regarding how to use the various elements of the kit, which instructions may comprise one or more elements of the method as defined herein.

Prior to applying the method or using the kit according to the present invention, a user should advantageously remove all make-up from the skin, to ensure good adherence and effective application of both the protective composition and the depilatory composition.

The method according to the first aspect of the invention comprises the step of applying the above-defined protective composition to an area of skin on which unwanted hair is growing. The area of skin may be located on any part of the human body, but is preferably on the face, more preferably on an area of skin adjacent to the vermillion lip and more preferably still on an area above the upper vermillion lip.

Advantageously, the protective composition is not just applied to the area to be depilated, but also to an immediately juxtaposing area thereabout (that is, the protective composition is applied to an area of skin which is greater than just the area which is to be depilated).

Advantageously, the user will apply from 0.3 - 2mg of protective composition per square centimetre of skin, preferably from 0.4 - 1mg/$cm^2$, more preferably from 0.4 to 0.7mg/$cm^2$.

Following application, the protective composition is advantageously massaged into the skin. Preferably, massaging is effected for at least 10 seconds, and, more preferably, massaging is effected as a circular motion. Without wishing to be bound by theory, it is believed that the protective composition may trap hair within it thereby shielding it from the to-be-applied depilatory composition; massaging may help to release the hairs from the skin and ensure improved access thereto by the depilatory composition.

The method according to the first aspect of the invention comprises the subsequent step of applying the above-defined depilatory composition to an area of skin on which unwanted hair is growing and to which protective composition has already been applied. Advantageously, the user will apply a layer of depilatory composition which is from 0.1mm to 5mm, preferably from 0.3 to 3mm, more preferably from 0.5 to 2mm in thickness.

Subsequently, according to the method of the first aspect of the invention, the depilatory composition is advantageously left in place for at least 1 minute, preferably from 1 to 10 minutes, more preferably from 3 to 10 minutes, depending on the thickness of the hair and the hair removal efficacy of the depilatory composition (which, in turn, is dependent upon the concentration of keratin reducing agent in the depilatory composition).

Subsequently, according to the method of the first aspect of the invention, the protective composition and the depilatory composition are advantageously removed. This may be achieved using one or more of a cotton wool ball, pad or wand, a tissue, a cloth, or a tool, such as a spatula or a scraper. Advantageously, the skin from which hair has been removed is then rinsed with water.

In an advantageous subsequent step, a post-treatment skin care composition may be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

Differential Scanning Calorimetry (DSC) Melting Method

This method is the American Oil Chemists' Society Method Cj 1-94, as reapproved in 2009 and it determines the "onset temperature" (that is the temperature of onset of melting) of oils and fats by differential scanning calorimetry (DSC).

Apparatus

1. Aluminum capsules.

2. DSC instrument, capable of holding temperature at -60°C and achieving a temperature of 80°C.

Reagents

1. Indium, powder—60 mesh, 99.999%, such as Aldrich Chemical Co., Milwaukee, WI 53233, or equivalent.

2. *n*-Decane, 99+%, such as Aldrich Chemical Co., Milwaukee, WI 53233, or equivalent.

3. Methyl stearate, 99%, such as Aldrich Chemical Co., Milwaukee, WI 53233, or equivalent.

Procedure

1. Standardization of equipment-Proceed with the normal standardization using both indium and n-decane as reference standards. Follow instrument manual for adjustment to lock onto these two reference points and flatten the baseline slope as much as possible when empty pans are analyzed. Analyze the secondary standard (methyl stearate). Weigh 5 mg of the standard into the same kind of pan which will be used for the test portion (if hermetically sealed, it may be reused at a later date). Use the method sequence in Procedure, 2-7 to obtain the melting point onset (because of the high purity, only a 2 min hold is necessary for the standard after crystallization). Be certain that the heating rate during the definitive heating pattern is at 5°C/min. The melting point onset should be within ±2.00°C of 36.5°C. If not, recheck calibration.
*Note*-be certain to use identical capsules for the test portion as those used for reference standards and the instrument blank reference.

2. Melt each test portion completely and weigh 7 ± 0.200 mg of each test portion into the same kind of capsule used for the blank and reference samples (aluminum) and seal to minimize oxidation and other changes.

3. Place capsules in DSC at room temperature.

4. Heat rapidly to 80°C and hold for 10 min.

5. Cool to -60°C at 10°C/min and hold for 30 min.

6. Heat to 80°C at 5°C/min.

7. Use the baseline obtained for an empty capsule analysis from the final melt segment of the program to define the position of the baseline under the sample peaks. Overlay the final melting curve of the test portion over the curve for the empty capsule with a flexible ruler or other curve guide to define the baseline of the test portion back to where it intersects the initial deviation of the melting curve from its baseline. The baseline beneath the test portion should be a continuation of the baseline where there are no sample components present. If a shift has occurred in the heat capacity of the test portion after the melt, it will be evident relative to the baseline of the empty capsule. Have the instrument calculate the sigmoid baseline if it can, or connect the end of the peak point with the last point in which the test portion was in conjunction with the baseline of the empty capsule.

Results

Determine the onset temperature in °C, which, if not computer generated, is an extrapolation to baseline of the steepest slope of the principal peak.

Franz cell method

Principle and Scope:

This method is applicable for using Franz cell apparatus for the *in-vitro* assessment of penetration of thioglycolic acid (TGA) and its salts through a skin mimic after the application of a depilatory composition following pre-treatment with a protective composition.

Penetrated TGA is quantified using Reverse Phase High Performance (or Pressure) Liquid Chromatography (RP-HPLC) with external standard quantitation at 240nm.

Method

Reference is made to Figure 1 and to the reference numerals therein:

1. Prepare the Vitro-Skin (IMS Vitro-Skin®, Catalogue number: P&G1013, made by IMS Inc., Portland, Maine, USA) samples by cutting 8x6.2cm segments and placing them textured side up on the racks into a hydration chamber (manufactured & sold by IMS) containing a 14.7% glycerol solution. The hydration chamber should be sealed and the vitro-skin left to hydrate at room temperature and a humidity of 80.4% $\pm$3.5% for 24 hours.

2. Prepare the receptor solution for the Franz-cell by mixing 1.90ml formic acid (98%wt+ Fluka, by Sigma Aldrich, or equivalent), 30ml acetonitrile (RP-HPLC grade) and 968.1ml water (RP-HPLC grade). Set up the static Franz cell (Permegear or equivalent, 15mm diameter unjacketed cell with a 12ml receptor volume) by clamping it in place over suitable stirrer plates (not shown) and add a small stirrer bar (6) to each cell, fill the receptor cell (2) to the brim with the required amount of receptor solution.

3. Once hydrated, remove a sheet of vitro-skin from the hydration chamber and lay textured side up on a clean flat surface then dose 100$\mu$l (~2mg/$cm^2$) of protective composition (not shown) onto the vitro-skin and spread evenly over the surface by rubbing for 30 seconds with a gloved finger.

4. Using a scalpel blade cut the vitro-skin segment (3) into two equal sections, each large enough to completely cover the top of the cell. Place the relevant size o-ring (5) (22mm, for the specified Franz-cell) onto each section of the vitro skin and dose to 150mg/$cm^2$ of depilatory composition (4) ("Veet Normal Skin Hair Removal Cream" or an equivalent (an equivalent being a composition comprising 3.7%wt thioglycolic acid)) into the centre then, using a glass rod, evenly spread the cream around the inside of the o-ring (5). Using tweezers pick up the vitro-skin segment and place the vitro-skin segment, depilatory and o-ring centrally over the receptor cell (2), place donor cell (1) over the top and clamp in place. Turn on stirrer plate and start 10 minute countdown timer. After 10 minutes; turn off stirrer and remove the clamp, donor cell (1) and vitro-skin segment and place the receptor solution in a suitable container for analysis.

5. A reference sample should also be run without protective composition treatment on the vitro-skin. Remove a sheet of vitro-skin from the hydration chamber and lay textured side up on a clean flat surface. Repeat step 4 of the protocol to produce the reference sample.

Sample Analysis

For RP-HPLC analysis, prepare a 50mM Formic acid (98%+ Fluka) solution and mix 970ml of this solution with 30ml acetonitrile (HPLC grade) to act as a mobile phase during the analysis.

A reference standard solution should be made with a concentration of Calcium Thioglycolate Trihydrate of 0.94 mg/ml.

Install a Waters Atlantis T3 3$\mu$m 4.6 x 50mm column into the HPLC (although any silica- based $C_{18}$ reversed phase RP-HPLC column may be used), and ensure all solvent lines for the RP-HPLC are primed and free of leaks. Allow the mobile phase to circulate through the system for 25 minutes at 0.7mL/Min in order to equilibrate the column. Detection of the thioglycolic acid is via UV spectroscopy.

The RP-HPLC conditions are as follows:

- Injection volume: 20 $\mu$L
- Mobile phase flow rate: 0.70 ml/min

- Run time: 10 minutes
- UV Detection wavelength: 240 nm
- Column temperature: 35°C
- UV sampling rate: ≥ 5 per second
- Retention time: Thioglycolic Acid ~ 2.5 min

Calculations:

Calculate the concentration of Thioglycolic Acid in the sample

$$\text{concentration (mg/ml)} = \frac{\textbf{weight of std (mg) x purity}}{\textbf{25}} \times \frac{\textbf{3}}{\textbf{25}}$$

Calculate the concentration of thioglycolic acid in the sample using the following formula:

$$\text{concentration (mg/ml)} = \textbf{A/B} \times \textbf{C} \times \textbf{E/F}$$

Where,

A = Peak Area of Thioglycolic Acid Sample
B = Average Peak Area of Thioglycolic Acid Standard
C = Thioglycolic Acid final STD concentration in mg/ml (0.94 mg/ml)
E = Molecular weight of Thioglycolic acid (92.12g/mol)
F = Molecular weight of Calcium thioglycolate (184.23g/mol)

The efficacy of the barrier provided by the protective composition (resistance to TGA penetration) can be calculated as a percentage decrease in TGA in the receptor solution:

$$\%\text{reduction} = \frac{\text{concentration without barrier* – concentration with barrier*}}{\text{concentration without barrier*}} \times 100$$

*protective composition

For example, if TGA in solution without protective composition = 75μg/ml and TGA in solution with barrier = 15 μg/ml

$$\%\text{reduction} = \frac{75 - 60}{75} \times 100 = \underline{\textbf{85\%}}$$

A reduction of TGA penetration of 45% or more is believed to correlate to a significant and user-noticeable reduction in irritation.

Examples

Inventive Example 1

Premix, Oil Phase

| INCI | Trade Name | %w/w |
|---|---|---|
| Sucrose Polycottonseedate* | Sefa Cottonate | 38.160 |
| Cera Alba | Beeswax | 1.800 |
| Propyl Paraben | Propyl Paraben | 0.040 |
| *oil | | |

Main Mixture, Water Phase

| INCI | Trade Name | %w/w |
|---|---|---|
| Purified Water | Deionised Water | 59.600 |
| Acrylates/C 10-30 Alkyl Acrylate Crosspolymer | Pemulen TR-2 | 0.400 |

Making Instructions

- Add all ingredients from Premix into a premix tank (PMT) and heat to 75°C, mixing using an overhead stirrer set to 200rpm. Ensure enough agitation to stop the wax from settling to the bottom of the PMT.
- Once the ingredients within the PMT have reached 75°C, maintain stirring and leave the PMT at 75°C for 45 minutes to ensure that the oil phase has fully melted.
- Cool the PMT to 25°C whilst mixing using the overhead stirrer set to 60rpm. Ensure that aeration of the PMT mixture is avoided.
- Place all ingredients from the Main Mixture into the main mixing tank (MMT).
- Add the mixture from the PMT into the MMT. Using a high shear mixing device (such as a Silverson Laboratory Mixer available from Silverson Machines), mix the contents of the MMT for 5 minutes at ~7000rpm to ensure full emulsification of the phases.
- Transfer product from the MMT to a suitable storage container.

The comporsition of Inventive Example 1 was tested using a Franz Cell according to the above-defined method and gave a percentage reduction in thioglycolate penetration of 83%.

The compositions of Inventive Examples 2-6 were made in an analogous fashion to Inventive Example 1. The compositions were then tested using the Franz Cell method defined above.

| Example | Protective Composition | % Reduction in Thioglycolic Acid Penetration According to the Franz Cell Method |
|---|---|---|
| Inventive Example 2 | 49.6% Water<br>47.7% Sefa Cottonate<br>2.25% Beeswax<br>0.4% Acrylates/C10-30 Alkyl Acrylate Crosspolymer<br>0.05% Propyl Paraben | 97 |

(continued)

| Example | Protective Composition | % Reduction in Thioglycolic Acid Penetration According to the Franz Cell Method |
|---|---|---|
| Inventive Example 3 | 29.6% Water<br>60% Mineral Oil<br>10% Beeswax<br>0.4% Acrylates/C10-30 Alkyl Acrylate Crosspolymer | 98 |
| Inventive Example 4 | 39.6% Water<br>56% Mineral Oil<br>4% Carnauba<br>0.4% Acrylates/C10-30 Alkyl Acrylate Crosspolymer | 80 |
| Comparative Example 1 | 76.95% Water<br>10% Isohexadecane<br>10% Beeswax<br>1.75% Polyacrylamide & C13-14 Isoparafin (Sepigel 305)<br>1% Stearyl Alcohol<br>0.3% Cetearyl Alcohol & Cetearyl Glucoside | 16.0 |
| Comparative Example 2 | 46.95% Water<br>30% Isohexadecane<br>20% Beeswax<br>1.75% Polyacrylamide & C13-14 Isoparafin (Sepigel 305)<br>1% Stearyl Alcohol<br>0.3% Cetearyl Alcohol & Cetearyl Glucoside | 15.0 |
| Comparative Example 3 | 100% mineral oil | 25.0 |
| Comparative Example 4 | 100% Sunflower Seed oil | 10.8 |
| Comparative Example 5 | 100% olive oil | 0.0 |

As demonstrated by the Franz Cell data, oils, on their own (see Comparative Examples 3,4 and 5) and emulsions comprising a hydrophilic continuous phase (and hydrophobic dispersed phase) stabilized by traditional surfactants (see Comparative Examples 1 and 2) provide little to no barrier to prevent thioglycolic acid penetration, whereas the protective compositons comprising emulsions according to the invention (see Inventive Examples 1-4) present a dramatically superior barrier to penetration.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

**1.** A method of removing hair from skin comprising the steps of:

(a) applying a protective composition to an area of skin on which unwanted hair is growing, wherein the protective

composition comprises a sterically stabilised emulsion comprising a hydrophilic continuous phase, a hydrophobic dispersed phase and a steric emulsifier, being an amphipathic polymer which comprises a hydrophilic backbone and hydrophobic side chains wherein the steric emulsifier comprises

1) a carboxylic acid copolymer comprising a monomeric mixture, the monomers being selected from the group consisting of acrylic acid, methacrylic acid and ethacrylic acid, comprising from 1% to 3.5% by weight of the carboxylic acid copolymer of an acrylate ester of the formula:

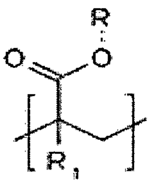

wherein $R_1$ is hydrogen, methyl or ethyl and R is an alkyl radical containing 10 to 30 carbon atoms; 95.9% to 98.8% by weight of the carboxylic acid copolymer of an acrylate ester according to the above formula, where R and $R_1$ are hydrogen, methyl or ethyl
and 0.1 to 0.6%, by weight of the carboxylic acid copolymer, of a polymerizable cross-linking polyalkenyl polyether of a polyhydric alcohol containing more than one alkenyl ether group per molecule, wherein the parent polyhydric alcohol contains at least 3 carbon atoms and at least 3 hydroxyl groups, or

2) alkyl substituted hydroxyethyl cellulose ether copolymer of the structure

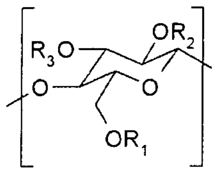

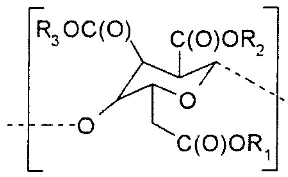

whereby a long chain alkyl modifier group having 8 to 25 carbon atoms is attached to the cellulose ether substrate via an ether or ester linkage, the remaining R-groups being either H, methyl or ethyl, the monomer containing the long chain hydrocarbon group is present in an amount of 0.1% to 4.0% by weight of the copolymer, with the remaining monomers having $R_1$, $R_2$ and $R_3$ groups of H, methyl and

(b) applying a depilatory composition to the area of skin to which the protective composition has been applied, the depilatory composition comprising a keratin reducing agent, the keratin reducing agent comprising at least one thioglycolate salt or thioglycolic acid

**2.** The method of claim 1, wherein the protective composition comprises from 0.01 % to 5%, preferably from about 0.05% to about 3%, more preferably from 0.05% to 1% of steric emulsifier by weight of the protective composition.

**3.** The method of claim 1 or 2, wherein the protective composition comprises 15% to 80%, preferably, 20% to 70%, more preferably 25% to 65% hydrophilic continuous phase by weight of the protective composition.

**4.** The method of any of claims 1 to 3, wherein the protective composition comprises 20% to 85%, preferably, 20% to 75%, more preferably 35% to 70% hydrophobic dispersed phase by weight of the protective composition.

**5.** The method of any of claims 1 to 4, wherein the hydrophobic dispersed phase comprises wax.

**6.** The method of claim 5, wherein the protective composition comprises 0.75% to 15%, preferably 1% to 10%, more preferably 1% to 8% wax by weight of the protective composition.

**7.** The method of claim 5 or 6, wherein the wax comprises natural wax, synthetic wax, silicone wax, or mixtures thereof.

**8.** The method of any preceding claim, wherein the hydrophobic continuous phase comprises silicone oil, preferably

dimethicone having a viscosity of 1 to 10,0000 centistoke, preferably 5 to 1000 centistoke, more preferably 25 to 500 centistoke.

**9.** The method of any preceding claim, comprising the following additional step between step (a) and step (b):

(a1) massaging the protective composition into the skin for at least 10 seconds.

**10.** The method of any preceding claim, wherein the thioglycolate salt comprises potassium or calcium thioglycolate, or mixtures thereof.

**11.** A depilatory kit comprising:

(a) a protective composition comprising a sterically stabilised emulsion comprising a hydrophilic continuous phase, a hydrophobic dispersed phase and a steric emulsifier, being an amphipathic polymer which comprises a hydrophilic backbone and hydrophobic side chains as defined in claim 1;
(b) a depilatory composition comprising an effective amount of a keratin reducing agent, the keratin reducing agent comprising at least one thioglycolate salt or thioglycolic acid.

**12.** The depilatory kit of claim 11, additionally comprising a tool, such as a scraper or a spatula, or a wipe.

**Patentansprüche**

**1.** Verfahren zum Entfernen von Haaren auf der Haut, das folgende Schritte umfasst:

(a) das Auftragen einer Schutzzusammensetzung auf eine Hautfläche mit unerwünschtem Haarwuchs, wobei die Schutzzusammensetzung eine sterisch stabilisierte Emulsion umfasst, welche eine hydrophile kontinuierliche Phase, eine hydrophobe disperse Phase und einen sterischen Emulgator umfasst, der ein amphipatisches Polymer ist, das ein hydrophiles Grundgerüst und hydrophobe Seitenketten umfasst, wobei der sterische Emulgator Folgendes umfasst:

1) ein Carbonsäurecopolymer, das eine monomere Mischung umfasst, wobei die Monomere ausgewählt sind aus der Gruppe bestehend aus Acrylsäure, Methacrylsäure und Ethacrylsäure, umfassend zu 1 Gew.-% bis 3,5 Gew.-% des Carbonsäurecopolymers einen Acrylatester der folgenden Formel:

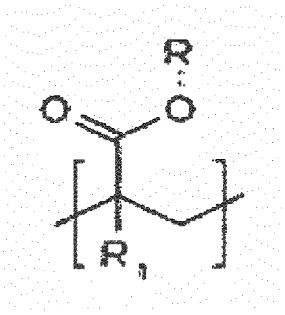

worin $R_1$ Wasserstoff, Methyl oder Ethyl ist und R ein Alkylrest ist, der 10 bis 30 Kohlenstoffatome enthält, zu 95,9 Gew.-% bis 98,8 Gew.-% des Carbonsäurecopolymers einen Acrylatester nach der vorstehenden Formel, worin R und $R_1$ Wasserstoff, Methyl oder Ethyl sind,
und zu 0,1 bis 0,6 Gew.-% des Carbonsäurecopolymers einen polymerisierbaren vernetzenden Polyalkenylpolyether eines Polyols, das mehr als eine Alkenylethergruppe pro Molekül enthält, wobei das Stammpolyol mindestens 3 Kohlenstoffatome und mindestens 3 Hydroxylgruppen enthält, oder

2) alkylsubstituiertes Hydroxyethylcelluloseethercopolymer der folgenden Struktur:

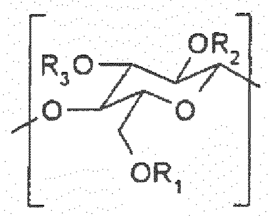

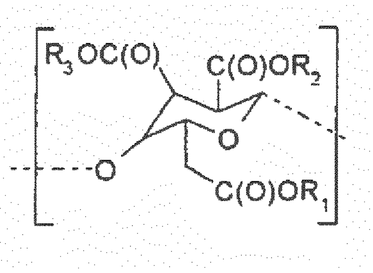

wobei eine langkettige Alkylmodifikatorgruppe mit 8 bis 25 Kohlenstoffatomen an dem Cellulose-Ether-Substrat über eine Ether- oder Esterbindung angebunden ist, wobei die restlichen R-Gruppen entweder H, Methyl oder Ethyl sind, wobei das die langkettige Kohlenwasserstoffgruppe enthaltende Monomer in einem Anteil von 0,1 Gew.-% bis 4,0 Gew.-% des Copolymers vorhanden ist, wobei die restlichen Monomere $R_1$-, $R_2$- und $R_3$-Gruppen an H, Methyl aufweisen, und

(b) das Auftragen einer Depilierzusammensetzung auf die Hautfläche, auf die die Schutzzusammensetzung aufgetragen wurde, wobei die Depilierzusammensetzung ein Keratinreduktionsmittel umfasst, wobei das Keratinreduktionsmittel mindestens ein Thioglycolatsalz oder Thioglycolsäure umfasst.

**2.** Verfahren nach Anspruch 1, wobei die Schutzzusammensetzung zu 0,01 Gew.-% bis 5 Gew.-%, vorzugsweise etwa 0,05 Gew.-% bis etwa 3 Gew.-%, mehr bevorzugt zu 0,05 Gew.-% bis 1 Gew.-% der Schutzzusammensetzung einen sterischen Emulgator umfasst.

**3.** Verfahren nach Anspruch 1 oder 2, wobei die Schutzzusammensetzung zu 15 Gew.-% bis 80 Gew.-%, vorzugsweise zu 20 Gew.-% bis 70 Gew.-%, mehr bevorzugt zu 25 Gew.-% bis 65 Gew.-% der Schutzzusammensetzung hydrophile kontinuierliche Phase umfasst.

**4.** Verfahren nach einem der Ansprüche 1 bis 3, wobei die Schutzzusammensetzung zu 20 Gew.-% bis 85 Gew.-%, vorzugsweise zu 20 Gew.-% bis 75 Gew.-%, mehr bevorzugt zu 35 Gew.-% bis 70 Gew.-% der Schutzzusammensetzung hydrophobe disperse Phase umfasst.

**5.** Verfahren nach einem der Ansprüche 1 bis 4, wobei die hydrophobe disperse Phase Wachs umfasst.

**6.** Verfahren nach Anspruch 5, wobei die Schutzzusammensetzung zu 0,75 Gew.-% bis 15 Gew.-%, vorzugsweise zu 1 Gew.-% bis 10 Gew.-%, mehr bevorzugt zu 1 Gew.-% bis 8 Gew.-% der Schutzzusammensetzung Wachs umfasst.

**7.** Verfahren nach Anspruch 5 oder 6, wobei das Wachs natürliches Wachs, synthetisches Wachs, Silikonwachs oder Mischungen davon umfasst.

**8.** Verfahren nach einem der vorstehenden Ansprüche, wobei die hydrophobe Dispersionsphase Silikonöl umfasst, vorzugsweise Dimethicon mit einer Viskosität von 1 bis 10.000 $mm^2/s$ (1 bis 10.0000 Centistoke), vorzugsweise 5 bis 1000 $mm^2/s$ (5 bis 1000 Centistoke), mehr bevorzugt 25 bis 500 $mm^2/s$ (25 bis 500 Centistoke).

**9.** Verfahren nach einem der vorstehenden Ansprüche, das zwischen Schritt (a) und Schritt (b) den folgenden zusätzlichen Schritt umfasst:

(al) das Einmassieren der Schutzzusammensetzung in die Haut mindestens 10 Sekunden lang.

**10.** Verfahren nach einem der vorstehenden Ansprüche, wobei das Thioglycolatsalz Kalium- oder Calciumthioglycolat oder Mischungen davon umfasst.

**11.** Enthaarungsset, das Folgendes umfasst:

(a) eine Schutzzusammensetzung, die eine sterisch stabilisierte Emulsion umfasst, die eine hydrophile kontinuierliche Phase, eine hydrophobe disperse Phase und einen sterischen Emulgator umfasst, der ein amphipatisches Polymer ist, das ein hydrophiles Grundgerüst und hydrophobe Seitenketten wie in Anspruch 1 definiert umfasst,

(b) eine Depilierzusammensetzung, die eine wirksame Menge an Keratinreduktionsmittel umfasst, wobei das Keratinreduktionsmittel mindestens ein Thioglycolatsalz oder Thioglycolsäure umfasst.

**12.** Enthaarungsset nach Anspruch 11, das zusätzlich ein Werkzeug, wie einen Schaber oder Spatel oder ein Wischtuch, umfasst.

## Revendications

**1.** Procédé d'élimination de poils de la peau, comprenant les étapes consistant à :

(a) appliquer une composition protectrice sur une zone de peau sur laquelle poussent des poils indésirables, dans lequel la composition protectrice comprend une émulsion stériquement stabilisée comprenant une phase continue hydrophile, une phase dispersée hydrophobe et un émulsifiant stérique, étant un polymère amphipathique qui comprend un squelette hydrophile et des chaînes latérales hydrophobes dans lequel l'émulsifiant stérique comprend

1) un copolymère d'acide carboxylique comprenant un mélange monomère, les monomères étant choisis dans le groupe constitué d'acide acrylique, acide méthacrylique et acide éthacrylique,
comprenant de 1 % à 3,5 % en poids du copolymère acide carboxylique d'un ester acrylate de formule :

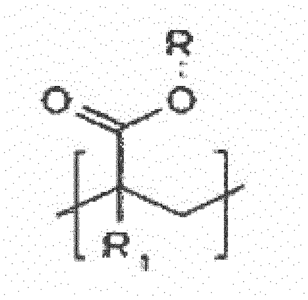

dans laquelle $R_1$ est hydrogène, méthyle ou éthyle et R est un radical alkyle contenant 10 à 30 atomes de carbone ;
95,9 % à 98,8 % en poids du copolymère acide carboxylique d'un ester acrylate selon la formule ci-dessus, où R et $R_1$ sont hydrogène, méthyle ou éthyle
et 0,1 à 0,6 % en poids du copolymère acide carboxylique, d'un polyéther polyalcényle de réticulation polymérisable d'un alcool polyhydrique contenant plus d'un groupe alcényl-éther par molécule, dans lequel l'alcool polyhydrique parent contient au moins 3 atomes de carbone et au moins 3 groupes hydroxyle, ou

2) un copolymère hydroxyéthylcellulose éther à substitution alkyle de structure

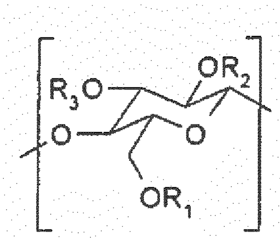

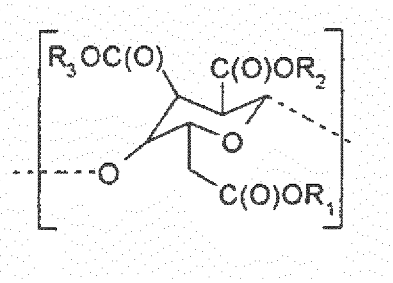

dans lequel un groupe modifiant alkyle à longue chaîne possédant 8 à 25 atomes de carbone est fixé au substrat éther de cellulose par l'intermédiaire d'une liaison éther ou ester, les groupes R restants étant ou H, ou méthyle ou éthyle, le monomère contenant le groupe hydrocarboné à longue chaîne est présent en une quantité de 0,1 % à 4,0 % en poids du copolymère, les monomères restants possédant des groupes $R_1$, $R_2$ et $R_3$ de H, méthyle et

(b) appliquer une composition dépilatoire sur la zone de peau sur laquelle la composition protectrice a été appliquée, la composition dépilatoire comprenant un agent réducteur de kératine, l'agent réducteur de kératine comprenant au moins un sel thioglycolate ou de l'acide thioglycolique.

**2.** Procédé selon la revendication 1, dans lequel la composition protectrice comprend de 0,01 % à 5 %, de préférence d'environ 0,05 % à environ 3 %, plus préférablement de 0,05 % à 1 % d'émulsifiant stérique en poids de la composition protectrice.

**3.** Procédé selon la revendication 1 ou 2, dans lequel la composition protectrice comprend 15 % à 80 %, de préférence, 20 % à 70 %, plus préférablement 25 % à 65 % de phase continue hydrophile en poids de la composition protectrice.

**4.** Procédé selon l'une quelconque des revendications 1 à 3, dans lequel la composition protectrice comprend 20 % à 85 %, de préférence, 20 % à 75 %, plus préférablement 35 % à 70 % de phase dispersée hydrophobe en poids de la composition protectrice.

**5.** Procédé selon l'une quelconque des revendications 1 à 4, dans lequel la phase dispersée hydrophobe comprend une cire.

**6.** Procédé selon la revendication 5, dans lequel la composition protectrice comprend 0,75 % à 15 %, de préférence 1 % à 10 %, plus préférablement 1 % à 8 % de cire en poids de la composition protectrice.

**7.** Procédé selon la revendication 5 ou 6, dans lequel la cire comprend une cire naturelle, une cire synthétique, une cire de silicone, ou leurs mélanges.

**8.** Procédé selon l'une quelconque des revendications précédentes, dans lequel la phase continue hydrophobe comprend une huile de silicone, de préférence de la diméthicone ayant une viscosité de 1 à 10 000 $mm^2/s$ (1 à 10 0000 centistokes), de préférence 5 à 1000 $mm^2/s$ (5 à 1000 centistokes), plus préférablement 25 à 500 $mm^2/s$ (25 à 500 centistokes).

**9.** Procédé selon l'une quelconque des revendications précédentes, comprenant l'étape supplémentaire suivante entre l'étape (a) et l'étape (b) :

(al) masser la composition protectrice dans la peau pendant au moins 10 secondes.

**10.** Procédé selon l'une quelconque des revendications précédentes, dans lequel le sel thioglycolate comprend du thioglycolate de potassium ou de calcium, ou leurs mélanges.

**11.** Trousse dépilatoire comprenant :

(a) une composition protectrice comprenant une émulsion stériquement stabilisée comprenant une phase continue hydrophile, une phase dispersée hydrophobe et un émulsifiant stérique, étant un polymère amphipathique qui comprend un squelette hydrophile et des chaînes latérales hydrophobes selon la revendication 1 ;
(b) une composition dépilatoire comprenant une quantité efficace d'un agent réducteur de kératine, l'agent réducteur de kératine comprenant au moins un sel thioglycolate ou de l'acide thioglycolique.

**12.** Trousse dépilatoire selon la revendication 11, comprenant en outre un outil, tel qu'un racloir ou une spatule ou une lingette.

Fig.1.

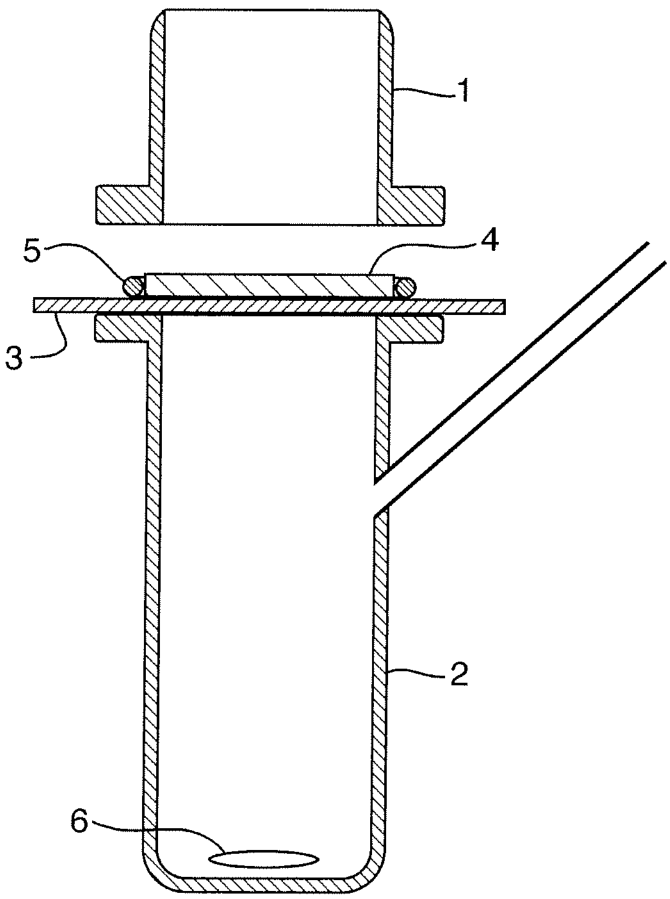

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4401663 A
- US 4424205 A
- US 20040219118 A


### Non-patent literature cited in the description


- United States' Pharmacopeia (USP). *General Notices,* vol. 2, Xvii
- United States' Pharmacopeia (USP). *General Notices,* vol. 2, Xvii
- Hydrophilic-Lipophilic Balance. The HLB System, A Time-Saving Guide to Emulsifier Selection. ICI Americas Inc, 1984
- The Encyclopaedia of Polymers and Thickeners for Cosmetics. Department of Polymer Science